(19) Europäisches Patentamt
European Patent Office
Office européen des brevets

(11)  EP 1 617 876 B1

(12)  **EUROPEAN PATENT SPECIFICATION**

(45) Date of publication and mention
of the grant of the patent:
**14.05.2014 Bulletin 2014/20**

(21) Application number: **04727587.0**

(22) Date of filing: **15.04.2004**

(51) Int Cl.:
**A61K 51/04** (2006.01)    **A61P 35/00** (2006.01)

(86) International application number:
**PCT/GB2004/001654**

(87) International publication number:
**WO 2004/091668 (28.10.2004 Gazette 2004/44)**

(54) **THORIUM-227 FOR USE IN RADIOTHERAPY OF SOFT TISSUE DISEASE**

THORIUM-227 ZUR RADIOTHERAPIE VON WEICHGEWEBE-ERKRANKUNGEN

THORIUM 227 UTILISABLE EN RADIOTHERAPIE POUR TRAITER UNE MALADIE DES TISSUS MOUS

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **15.04.2003 GB 0308731**

(43) Date of publication of application:
**25.01.2006 Bulletin 2006/04**

(73) Proprietor: **Algeta AS**
**0884 Oslo (NO)**

(72) Inventors:
• **LARSEN, Roy**
**N-0884 Oslo (NO)**
• **BRULAND, Oyvind**
**N-0884 Oslo (NO)**

(74) Representative: **Goddard, Christopher Robert**
**Dehns**
**St Bride's House**
**10 Salisbury Square**
**London**
**EC4Y 8JD (GB)**

(56) References cited:
WO-A-01/60417        WO-A-01/66155
WO-A-02/05859        WO-A-2004/043487
US-A1- 2001 008 625

• **MILENIC D E ET AL: "In vivo comparison of macrocyclic and acyclic ligands for radiolabeling of monoclonal antibodies with <177>Lu for radioimmunotherapeutic applications" NUCLEAR MEDICINE AND BIOLOGY, ELSEVIER SCIENCE PUBLISHERS, NEW YORK, NY, US, vol. 29, no. 4, May 2002 (2002-05), pages 431-442, XP004357346 ISSN: 0969-8051**
• **JACQUES V ET AL: "Kinetically and thermodynamically stable isomers of thorium chelates of polyaza polycarboxylic macrocycles", JOURNAL OF ALLOYS AND COMPOUNDS, ELSEVIER SEQUOIA, LAUSANNE, CH LNKD- DOI:10.1016/0925-8388 (94)90917-2, vol. 213-214, 1 October 1994 (1994-10-01), pages 286-289, XP022791336, ISSN: 0925-8388 [retrieved on 1994-10-01]**

**Description**

FIELD OF THE INVENTION

The present invention relates to a method of radiotherapy, more particularly such a method involving the use of thorium-227 in the treatment of soft tissue disease.

BACKGROUND OF THE INVENTION

Specific cell killing can be essential for the successful treatment of a variety of diseases in mammalian subjects. Typical examples of this are in the treatment of malignant diseases such as sarcomas and carcinomas. However the selective elimination of certain cell types can also play a key role in the treatment of other diseases, especially hyperplastic and neoplastic diseases.

The most common methods of selective treatment are currently surgery, chemotherapy and external beam irradiation. Targeted radionuclide therapy is, however, a promising and developing area with the potential to deliver highly cytotoxic radiation to unwanted cell types. The most common forms of radiopharmaceutical currently authorised for use in humans employ beta-emitting and/or gamma-emitting radionuclides. There has, however, been some interest in the use of alpha-emitting radionuclides in therapy because of their potential for more specific cell killing.

The radiation range of typical alpha emitters in physiological surroundings is generally less than 100 micrometers, the equivalent of only a few cell diameters. This makes these sources well suited for the treatment of tumours, including micrometastases, because little of the radiated energy will pass beyond the target cells and thus damage to surrounding healthy tissue might be minimised (see Feinendegen et al., Radiat Res 148:195-201 (1997)). In contrast, a beta particle has a range of 1 mm or more in water (see Wilbur, Antibody Immunocon Radiopharm 4: 85-96 (1991)).

The energy of alpha-particle radiation is high compared to beta particles, gamma rays and X-rays, typically being 5-8 MeV, or 5 to 10 times that of a beta particle and 20 or more times the energy of a gamma ray. Thus, this deposition of a large amount of energy over a very short distance gives $\alpha$-radiation an exceptionally high linear energy transfer (LET), high relative biological efficacy (RBE) and low oxygen enhancement ratio (OER) compared to gamma and beta radiation (see Hall, "Radiobiology for the radiologist", Fifth edition, Lippincott Williams & Wilkins, Philadelphia PA, USA, 2000). This explains the exceptional cytotoxicity of alpha emitting radionuclides and also imposes stringent demands on the level of control and study of alpha emitting radionuclide distribution necessary in order to avoid unacceptable side effects.

Very few alpha emitting radionuclides are currently considered useful for targeted molecular therapy (see for example Feinendegen Supra and Wilbur supra). In order to establish whether a particular nuclide is suitable, candidates must be carefully evaluated on the basis of their physical characteristics, chemical properties, and also the properties of the decay product(s) (i.e. their daughter nuclides). It is frequently the case that the daughter nuclides formed upon decay of the potentially therapeutic alpha-emitting radionuclide are also alpha-emitters and/or lead to further alpha-emitting isotopes when they in turn decay. It is therefore essential to consider the properties of potential daughter nuclides when assessing the therapeutic viability of an alpha-emitter.

Table 1 below shows the physical decay properties of the alpha emitters so far broadly proposed in the literature as possibly having therapeutic efficacy.

Table 1

| Candidate nuclide | $T_{1/2}$* | Clinically tested for |
|---|---|---|
| $^{225}$Ac | 10.0 days | Not tested |
| $^{211}$At | 7.2 hours | glioblastoma |
| $^{213}$Bi | 46 minutes | leukaemia |
| $^{223}$Ra | 11.4 days | skeletal metastases |
| $^{224}$Ra | 3.66 days | ankylosing spondylitis |
| * Half life | | |

So far, the main attention has been focused on $^{211}$At and $^{213}$Bi and these two nuclides have been explored in clinical immunotherapy trials.

Several of the radionuclides which have been proposed are short-lived, i.e. have half lives of less than 12 hours. Such short half lifes makes it difficult to produce and distribute radiopharmaceuticals based on these radionuclides in a commercial manner. Administration of a short-lived nuclide also increases the proportion of the radiation dose which will be emitted in the body before the target site is reached.

The two longer-lived nuclides $^{223}$Ra and $^{225}$Ac have more favourable half-lifes in this respect. Both these radionuclides have short-lived daughter products (mother and daughters emitting a combined total of four alphas), which could create a strong alpha cascade if decay of mother and daughters takes place at same location. If, however, the daughter nuclides are not contained in the target area, then these nuclides have the potential to release large quantities of damaging radiation into healthy tissues. There is also a significant and fundamental problem that the recoil of the daughter nucleus following alpha decay is highly energetic. (Release of a helium nucleus at around 2% of the speed of light imparts a very considerable amount of momentum to the remaining daughter nucleus).

The recoil energy from alpha-emission will in many cases cause the release of daughter nuclides from the position of decay of the parent. This recoil energy is sufficient to break many daughter nuclei out from the chemical environment which may have held the parent, e.g. where the parent was complexed by a ligand such as a chelating agent. This will occur even where the daughter is chemically compatible with, ie complexable by, the same ligand. Equally, where the daughter nuclide is a gas, particularly a noble gas such as radon, or is chemically incompatible with the ligand, this release effect will be even greater. When daughter nuclides have half-lives of more than a few seconds, they can diffuse away into the blood system, unrestrained by the complexant which held the parent. These free radioactive daughters can then cause undesired systemic toxicity.

Recently actinium-225 has attracted some attention; however research on this nuclide has been hampered by the low availability of source material. It has been shown that $^{225}$Ac could be linked to monoclonal antibodies and used for targeting of antigen containing tissues. In particular, WO 01/66155 discloses the use of alpha-radiator-chelator-antibody complexes of $^{225}$Ac in the treatment of cancer. However studies so far indicate that $^{225}$Ac labeled antibodies are highly toxic in animal experiments.

Other nuclides that have been mentioned as candidates for medical alpha-emitter therapy include $^{224}$Ra and $^{226}$Ra, ($T_{1/2}$ = 1600 years) which was used extensively in the early part of last century but was later abandoned because of negative long term effects including bone cancer. These two radium nuclides have radon daughters which are gaseous and diffuse rapidly away from the site occupied by the mother nuclide. There is also a general lack of suitable linkers for attachment of radium to a molecular targeting agent Additionally, the extremely long half-life of $^{226}$Ra is very problematic from a radiation safety and contamination hazard standpoint.

Most alpha-emitting radionuclides are thus considered generally unsuitable because of inappropriate half-lives or because their decay products are deemed as incompatible with medical use, e.g. because the daughter nuclides are bone-seeking (see Mausner, Med Phys 20, 503-509 (1993)). Thus, for example, radium, being a calcium analogue, is a particularly strong bone-targeter and if released from $^{227}$Th in vivo the daughter nuclide $^{223}$Ra is known (see Müller, Int J Radiat Biol 20: 233-243 (19971)) to accumulate undesirably in the skeleton where significant myelotoxicity can be expected.

According to Feinendegen et al ("Alpha-emitters for medical therapy" Second BiAnnual Workshop, Toronto, June 1998, US Dept. of Energy Report No. DOE/NE-0116), there are two therapeutic candidate radionuclides that decay via at least three alpha emitting progenies. These are $^{225}$Ac and $^{223}$Ra. Therapeutic studies in animal models with radioimmunoconjugates of actinium-225 ($T_{1/2}$=10.0 days) have indicated that injected dosages of 6.3 kBq (approximately 250 kBq/kg, assuming 25 g as the average animal weight) could strongly improve survival in mice bearing disseminated lymphoma xenografts. The actinium-225 series releases four alpha particles, the first three alpha emitters in the series having half-lives of less than 5 minutes, while the last alpha emitter in the series, bismuth-213, has a half-life of 46 minutes.

In vivo data demonstrating antitumor effects of $^{223}$Ra has not yet been presented in the literature. The advantage of this nuclide is the short-lived daughters, i.e. the daughters from $^{219}$Rn ($T_{1/2}$ = 3.96 seconds) release two more alphas within seconds, while the last alpha emitter in the series, $^{211}$Bi, besides being preceded by $^{211}$Pb ($T_{1/2}$ = 36.1 minutes), has a half life of 2.17 minutes and may difuse away from $^{223}$Ra. However, three of the four alpha-emitters will decay in the vicinity of the mother nuclide with $^{223}$Ra as well as $^{225}$Ac. This is in strong contrast to $^{227}$Th that has progenies emitting four alphas, all preceded by the 11.4 day half life daughter $^{223}$Ra. This long half-life of the $^{223}$Ra daughter is likely to cause almost complete translocalization of the progenies compared to the mother $^{227}$Th nuclide and thus considerable difficulties in controlling the site of these four alpha emissions and as a result a high likelihood of unwanted side effects.

However, thorium-227 ($T_{1/2}$ = 18.7 days) has recently been proposed (see WO 01/60417 and WO 02/05859) as a therapeutic radionuclide as long as a carrier system is used which allows the daughter nuclides to be retained by a closed environment. In one case, the radionuclide is disposed within a liposome and the substantial size of the liposome (as compared to recoil distance) helps retain daughter nuclides within the liposome. In the second case, bone-seeking complexes of the radionuclide are used which incorporate into the bone matrix and therefore restrict release of the daughter nuclides. These are potentially highly advantageous methods, but the administration of liposomes is not desirable in some circumstances and there are many diseases of soft tissue in which the radionuclides cannot be surrounded by a mineralised matrix so as to retain the daughter isotopes. Receptor conjugates with an antibody of folate and a

radionuclide such as $^{227}$Th are disclosed in US 2001/008625 and are considered suitable for use in the treatment of soft-tissue cancer forms.

In the absence of such specific means of retaining the radium daughters of thorium-227, the publicly available information regarding radium toxicity makes it clear that it is not possible to use thorium-227 as a therapeutic agent since the dosages required to achieve a therapeutic effect from thorium-227 decay would result in a highly toxic and possibly lethal dosage of radiation from the decay of the radium daughters, i.e. there is no therapeutic window.

There therefore remains a considerable need for the development of further radionuclide treatments of soft tissues which allow the therapeutic treatment of malignant and non-malignant disease with alpha-emitters without causing unacceptable side effects, particularly myelotoxicity.

The present inventors have now unexpectedly found that a therapeutic treatment window does exist in which a therapeutically effective amount of a targeted thorium-227 radionuclide can be administered to a subj ect (typically a mammal) without generating an amount of radium-223 sufficient to cause unacceptable myelotoxicity.

## SUMMARY OF THE INVENTION

Viewed from one aspect the present invention therefore provides the use of a therapeutically effective quantity of a soft-tissue targeting complex of thorium-227 and a complexing agent in the manufacture of a medicament for the treatment of soft-tissue disease in a mammalian subject, wherein said effective quantity is such that an acceptably non-myelotoxic quantity of radium-223 is generated *in vivo* by nuclear decay of the administered thorium-227 and wherein the thorium-227 is conjugated to a targeting moiety with bioaffinity, excluding bone-seekers, liposomes and folate conjugated antibodies or antibody fragments and wherein the therapeutically effective quantity of thorium-227 is at least 25 $kB_2$/kg.

Viewed from a further aspect the invention provides a pharmaceutical composition comprising a soft tissue targeting complex of thorium-227 and a complexing agent, together with at least one pharmaceutical carrier or excipient.

Viewed from a still further aspect the invention also provides a soft tissue targeting complex of thorium-227 and a complexing agent as defined in the claims.

So as to distinguish from non-radioactive thorium complexes, it should be understood that the thorium complexes and the compositions thereof claimed herein include thorioum-227 at greater than natural relative abundance, eg at least 20% greater. This does not affect the definition of the method of the invention where a therapeutically effective amount of thorium-227 is explicitly required.

Viewed from a yet still further aspect the invention also provides a kit for use in a method according to the invention, said kit comprising a solution of a soft tissue targeting complex of thorium-227 and a complexing agent together with instructions for the use of said solution in a method according to the invention.

## BRIEF DESCRIPTION OF THE ACCOMPANYING DRAWINGS

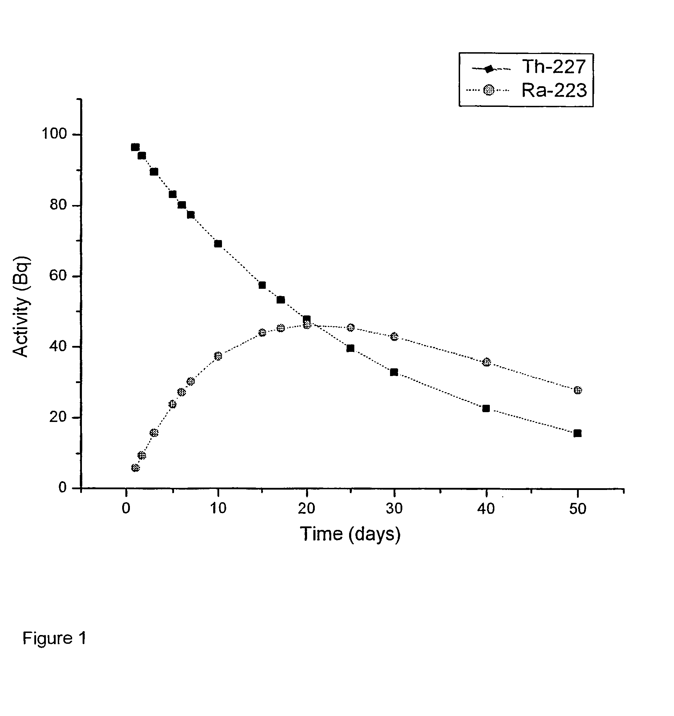

Figure 1 is a graph showing the drop with time of radioactivity due to thorium-227 decay and the resulting increase with time of radioactivity due to radium-223 decay following administration of a thorium-227 complex according to the invention.

## DETAILED DESCRIPTION OF THE INVENTION

By "soft tissue targeting" is meant herein that the substance in question, when in the form of a thorium complex, serves to bring the thorium to a soft tissue site at which its radioactive decay is desired. This may be by virtue of a component of the complexing agent which binds to cell-surface markers (e.g. receptors) present on disease affected cells or cells in the vicinity of disease affected cells (e.g. proteins more heavily expressed on diseased cell surfaces than on healthy cell surfaces or more heavily expressed on cell surfaces during periods of growth or replication than during dormant phases) or by virtue of a component which binds to a further soft tissue binding agent (in which case the further agent would be administered first as a "pathfinder" for the thorium complex). Alternatively, the targeting may be to an antigen which is associated with the target cells but not attached directly thereto. Such antigens will typically be in the matrix between the target cells and will thus be surrounded by diseased tissue. Examples of this are matrix antigens such as tenascin, which is associated with brain tumours but is expressed in the matrix between cells. Such matrix antigens can be targeted directly or with a preliminary binding agent, as discussed above.

The term "soft tissue" is used herein to indicate tissues which do not have a "hard" mineralised matrix. In particular, soft tissues as used herein may be any tissues that are not skeletal tissues. Correspondingly, "soft tissue disease" as used herein indicates a disease occurring in a "soft tissue" as used herein. The invention is particularly suitable for the treatment of cancers and "soft tissue disease" thus encompasses carcinomas, sarcomas, myelomas, lukemias, lymphomas and mixed type cancers occurring in any "soft" (i.e. non-mineralised) tissue, as well as other non-cancerous diseases of such tissue. Cancerous "soft tissue disease" includes solid tumours occurring in soft tissues as well as metastatic and micro-metastatic tumours. Indeed, the soft tissue disease may comprise a primary solid tumour of soft tissue and at least one metastatic tumours of soft tissue in the same patient. Alternatively, the "soft tissue disease" may consist of only a solid tumour or only metastases with the primary tumour being a skeletal disease.

As used herein, the term "acceptably non-myelotoxic" is used to indicate that, most importantly, the amount of radium generated is generally not sufficient to be directly lethal to the subject. It will be clear to the skilled worker, however, that the amount of marrow damage (and the probability of a lethal reaction) which will be an acceptable side-effect of such treatment will vary significantly with the type of disease being treated, the goals of the treatment regimen, and the prognosis for the subject. Although the preferred subjects for the present invention are humans, other mammals, particularly dogs, will benefit from the use of the invention and the level of acceptable marrow damage may also reflect the species of the subject. The level of marrow damage acceptable will generally be greater in the treatment of malignant disease than for non-malignant disease. One well known measure of the level of myelotoxicity is the neutrophil cell count and, in the present invention, an acceptably non-myelotoxic amount of $^{223}$Ra will typically be an amount controlled such that the neutrophil fraction at its lowest point (nadir) is no less than 10% of the count prior to treatment. Preferably, the acceptably non-myelotoxic amount of $^{223}$Ra will be an amount such that the neutrophil cell fraction is at least 20% at nadir and more preferably at least 30%. A nadir neutrophil cell fraction of at least 40% is most preferred.

In addition, $^{227}$Th containing compounds may be used in high dose regimens where the myelotoxicity of the generated $^{223}$Ra would normally be intolerable when stem cell support or a comparable recovery method is included. In such cases, the neutrophil cell count may be reduced to below 10% at nadir and exceptionally will be reduced to 5% or if necessary below 5%, providing suitable precautions are taken and subsequent stem cell support is given. Such techniques are well known in the art.

Thorium-227 is relatively easy to produce and can be prepared indirectly from neutron irradiated $^{226}$Ra, which will contain the mother nuclide of $^{227}$Th, i. e. $^{227}$Ac ($T_{1/2}$ = 22 years). Actinium-227 can quite easily be separated from the $^{226}$Ra target and used as a generator for $^{227}$Th. This process can be scaled to industrial scale if necessary, and hence the supply problem seen with most other alpha-emitters considered candidates for molecular targeted radiotherapy can be avoided.

Thorium-227 decays via radium-223. In this case the primary daughter has a half-life of 11.4 days. From a pure $^{227}$Th source, only moderate amounts of radium are produced during the first few days. However, the potential toxicity of $^{223}$Ra is higher than that of $^{227}$Th since the emission from $^{223}$Ra of an alpha particle is followed within minutes by three further alpha particles from the short-lived daughters (see Table 2 below which sets out the decay series for thorium-227).

Table 2

| Nuclide | Decay mode | Mean particle energy (MeV) | Half-life |
|---------|-----------|---------------------------|-----------|
| $^{227}$Th | α | 6.02 | 18.72 days |
| $^{223}$Ra | α | 5.78 | 11.43 days |
| $^{219}$Rn | α | 6.88 | 3.96 seconds |
| $^{215}$Po | α | 7.53 | 1.78 ms |
| $^{211}$Pb | β | 0.45 | 36.1 minutes |
| $^{211}$Bi | α | 6.67 | 2.17 minutes |
| $^{207}$Ti | β | 1.42 | 4.77 minutes |
| $^{207}$Pb | | | Stable |

Partly because it generates potentially harmful decay products, thorium-227 ($T_{1/2}$ = 18.7 days) has not been widely considered for alpha particle therapy.

The present inventors have established for the first time that thorium-227 may be administered in amounts sufficient to provide desirable therapeutic effects without generating so much radium-223 as to cause intolerable bone marrow supression.

Assuming the tumour cell killing effect will be mainly from thorium-227 and not from its daughters, the likely therapeutic dose of this isotope can be established by comparison with other alpha emitters. For example, for astatine-211, therapeutic doses in animals have been typically 2-10 MBq per kg. By correcting for half-life and energy the corresponding dosage for thorium-227 would be at least 36-200 kBq per kg of body weight. This would set a lower limit on the amount of $^{227}$Th that could usefully be administered in expectation of a therapeutic effect. This calculation assumes comparable retention of astatine and thorium. Clearly however the 18.7 day half-life of the thorium will most likely result in greater elimination of this isotope before its decay. This calculated dosage should therefore normally be considered to be the minimum effective amount. The therapeutic dose expressed in terms of fully retained $^{227}$Th (i.e. $^{227}$Th which is not eliminated from the body) will typically be at least 25 kBq/kg, preferably at least 36 kBq/kg and more preferably at least 75 kBq/kg, for example 100 kBq/kg or more. Greater amounts of thorium would be expected to have greater therapeutic effect but cannot be administered if intolerable side effects will result. Equally, if the thorium is administered in a form having a short biological half-life (ie the half life before elimination from the body still carrying the thorium), then greater amounts of the radioisotope will be required for a therapeutic effect because much of the thorium will be eliminated before it decays. There will, however, be a corresponding decrease in the amount of radium-223 generated. The above amounts of thorium-227 to be administered when the isotope is fully retained may easily be related to equivalent doses with shorter biological half-lives. Such calculations are given in Examples 1 and 2 below.

As an example, calculation of the amount of $^{227}$Th which is equivalent to a particular fully retained dose may be calculated by assuming negligible retention at the target site. In this case:

$$D_{ret} \approx \frac{D_{add}}{T_{Th}\left((T_{Bio})^{-1} + (T_{Th})^{-1}\right)}$$

wherein

$D_{add}$ is the administered dose;
$D_{ret}$ is the equivalent, fully retained dose;
$T_{Th}$, is the physical half-life of $^{227}$Th (18.7 days); and
$T_{Bio}$ is the biological half-life of the administered thorium complex.

The minimum effective dose of a thorium complex can thus readily be estimated. The biological half life moreover can be determined without having to use a radioactive form of the thorium complex.

If a radiolabeled compound releases daughter nuclides, it is important to know the fate, if applicable, of these radioactive daughter nuclide(s). With $^{227}$Th, the main daughter product is $^{223}$Ra, which is under clinical evaluation because of its bone seeking properties. Radium-223 clears blood very rapidly and is either concentrated in the skeleton or excreted via intestinal and renal routes (see Larsen, J Nucl Med 43(5, Supplement): 160P (2002)). Radium-223 released in vivo from $^{227}$Th may therefore not affect healthy soft tissue to a great extend. In the study by Müller in Int. J. Radiat. Biol. 20:233-243 (1971) on the distribution of $^{227}$Th as the dissolved citrate salt, it was found that $^{223}$Ra generated from $^{227}$Th in soft tissues was readily redistributed to bone or was excreted. The known toxicity of $^{223}$Ra, particularly to the bone marrow, is thus likely to be the limiting factor when $^{227}$Th is used *in vivo.*

The present inventors have established that $^{227}$Th complexes (e.g. chelate complexes) release $^{223}$Ra after decay of $^{227}$Th (see Example 6 below). This may be the result of nuclear recoil, or incompatible chelation, or a combination of factors. This is against what is accepted in the art as a desirable property for an alpha emitter (see for example Feinendegen et al. 1998, supra) in that the alpha emitter compound should retain any radioactive daughter nuclides in the parent chelate as an important safety characteristic.

According to data currently available in the art, the maximum tolerable dose of radium-223 can be expected to be in the range of 39 to 113 kBq/kg (see Example 7 below). It is accepted in the art that a realistic and conservative estimate of the toxic side effects of daughter isotopes must be adopted (see for example Finendagen (1998) supra) and thus a maximum of 39 kBq/kg of radium-223 would be considered acceptable. At any dose greater than this, the radium can be expected to become lethal to the subject, which of course must be considered unacceptable.

The generation of $^{223}$Ra in vivo will vary with the residence time of $^{227}$Th. In the case of 100% retention, 1 kBq of $^{227}$Th would generate a number of $^{223}$Ra atoms equivalent to an injected dose of 1.6 kBq of $^{223}$Ra, completely retained. Thus, a maximum tolerable dose of 39 kBq/kg of radium-223 is equivalent to an administration of 24.4 kBq/kg of completely retained $^{227}$Th. This is considerably below the minimum expected therapeutic dose of 36 kBq/kg, which was also estimated on the basis of complete retention (see discussion supra). If the retention of the thorium decreases, less radium will be generated per unit of thorium administered but the effectiveness of the thorium will be correspondingly reduced and so the dose must be increased. Thus, the expectation from the available evidence in the art was that the minimum amount of $^{227}$Th sufficient to provide any therapeutic benefit would be greater than the amount expected to cause lethal myelotoxicity. Therefore no therapeutic window for the administration of $^{227}$Th could have been seen to exist.

Significantly, the present inventors have now established that, in fact, a dose of at least 200 kBq/kg of $^{223}$Ra can be administered and tolerated in human subjects. These data are presented below in Example 8. Therefore, it can now be seen that, quite unexpectedly, a therapeutic window does exist in which a therapeutically effective amount of

$^{227}$Th (such as greater than 36 kBq/kg) can be administered to a mammalian subject without the expectation that such a subject will suffer an unacceptable risk of serious or even lethal myelotoxicity.

The amount of $^{223}$Ra generated from a $^{227}$Th pharmaceutical will depend on the biological half-life of the radiolabeled compound. The ideal situation would be to use a complex with a rapid tumor uptake, including internalization into tumor cell, strong tumor retention and a short biological half-life in normal tissues. Complexes with less than ideal biological half-life can however be useful as long as the dose of $^{223}$Ra is maintained within the tolerable level. The amount of radium -223 generated *in vivo* will be a factor of the amount of thorium administered and the biological retention time of the thorium complex. The amount of radium-223 generated in any particular case can be easily calculated by one of ordinary skill and examplary calculations are given in Examples 1 and 2 below. The maximum administrable amount of $^{227}$Th will be determined by this amount of radium generated *in vivo* and must be less than the amount that will produce an intolerable level of side effects, particularly myelotoxicity. This amount will generally be less than 300kBq/kg, particularly less than 200 kBq/kg and more preferably less than 170 kBq/kg (e.g less than 130 kBq/kg).

Thus, in the method of invention, the thorium complex is desirably administered at a thorium-227 dosage of 18 to 400 kBq/kg bodyweight, preferably 36 to 200 kBq/kg, (such as 50 to 200 kBq/kg) more preferably 75 to 170 kBq/kg, especially 100 to 130 kBq/kg. The thorium dosage, the complexing agent and the administration route will moreover desirably be such that the radium-223 dosage generated in vivo is less than 300 kBq/kg, more preferably less than 200 kBq/kg, still more preferably less than 150 kBq/kg, especially less than 100 kBq/kg. The above dose levels are preferably the fully retained dose of $^{227}$Th but may be the administered dose taking into account that some $^{227}$Th will be cleared from the body before it decays.

Where the biological half-life of the $^{227}$Th complex is short (e.g. less than 7 day, especially less than 3 days) significantly larger administered doses may be needed to provide the equivalent retained dose. Thus, for example, a fully retained dose of 150 kBq/kg is equivalent to a complex with a 5 day half-life administered at a dose of 711 kBq/kg, according to the equation set out herein above. By use of this equation, the equivalent administered dose for any of the above retained doses may be calculated from the biological clearance rate of the complex.

Since the decay of one $^{227}$Th nucleus provides one $^{223}$Ra atom, the retention and therapeutic activity of the $^{227}$Th will be directly related to the $^{223}$Ra dose suffered by the patient.

In a simplified calculation, the *in vivo* generation of $^{223}$Ra may be related to the amount of $^{227}$Th administered by assuming no significant retention in the target tissue. The maximum tolerable dose of $^{227}$Th may then be expressed as:

$$\frac{1.65 \times D_{add}}{T_{Th}\left((T_{Bio})^{-1} + (T_{Th})^{-1}\right)} < \text{Max Ra}$$

where:

$T_{Bio}$ is the biological half-life of the $^{227}$Th complex;
$T_{Th}$ is the physical half-life of $^{227}$Th (18.7 days);
$D_{add}$ is the activity of the administered $^{227}$Th complex (kBq/kg); and
Max Ra is the acceptably non-myelotoxic amount of $^{223}$Ra (kBg/kg) as discussed herein.

In a preferred embodiment, the present invention therefore provides a method for the treatment of soft tissue disease in a mammalian subject, said method comprising administering to said subject a therapeutically effective quantity of a soft tissue targeting complex of thorium-227 and a complexing agent, wherein said quantity is $D_{add}$ as calculated from formula I below, such that an acceptably non-myelotoxic quantity $D_{Ra}$ of radium-223 is generated *in vivo* by nuclear decay of the administered thorium-227;

$$D_{add} = \frac{D_{Ra} \times T_{Th}\left((T_{Bio})^{-1} + (T_{Th})^{-1}\right)}{1.65} \qquad (I)$$

wherein:

$T_{Bio}$ is the biological half-life of said soft tissue targeting complex of thorium-227 and a complexing agent;
$T_{Th}$ is the physical half-life of $^{227}$Th (18.7 days);
$D_{add}$ is the activity of the administered $^{227}$Th complex (kBq/kg); and
$D_{Ra}$ is the acceptably non-myelotoxic amount of $^{223}$Ra of, for example, 75 to 200 kBg/kg.

The biological half-life of any particular soft tissue targeting complex of thorium-227 and a complexing agent may be measured by known techniques using non-radioactive thorium or by measurement of, for example, characteristic gamma emissions using very low levels of thorium-227 in combination with non-radioactive thorium. From this formula, for example, it can be seen that a non- myelotoxic dose of 100 kBg/kg $^{223}$Ra is generated from a soft tissue targeting complex of thorium-227 and a complexing agent having a biological half-life of 10 days by an administered dose of [100 x 18.7 x (10$^{-1}$ $^+$ 18.7$^{-1}$)]/1.65 = 173 kBq/kg

It is obviously desirable to minimise the exposure of a subject to the $^{223}$Ra daughter isotope, unless the properties of this are usefully employed. In order to allow sufficient $^{227}$Th to be administered, however, it is necessary that a certain amount of radium be generated. This amount will be one which is sufficient to allow a therapeutically effective administration of $^{227}$Th and will generally be greater than the amount that would previously have been taken as the maximum acceptable in view of the expected $^{223}$Ra myelotoxicity (see Examples 7 and 8 below and the discussion supra). In particular, the amount of radium-223 generated *in vivo* will typically be greater than 40 kBq/kg, e.g. greater than 60 kBq/Kg. In some cases it will be necessary for the $^{223}$Ra generated *in vivo* to be more than 80 kBq/kg, e.g. greater than 100 or 115 kBq/kg.

Thorium-227 labelled complexes in appropriate carrier solutions may be administered intravenously, intracavitary (e.g. intraperitoneally), subcutaneously, orally or topically, as a single application or in a fractionated application regimen. Preferably the complexes will be administered as solutions by a parenteral route, especially intravenously or by an intracavitary route. Preferably, the compositions of the present invention will be formulated in sterile solution for parenteral administration.

Thorium-227 in the methods and products of the present invention can be used alone or in combination with other treatment modalities including surgery, external beam radiation therapy, chemotherapy, other radionuclides, or tissue temperature adjustment etc. This forms a further, preferred embodiment of the method of the invention.

According to this invention $^{227}$Th may be complexed by targeting complexing agents. Typically these will have a molecular weight from 100 g/mol to several million g/mol, and will preferably have affinity for a disease-related receptor and/or a suitable pre-administered receptor (e.g. biotin or avidin) bound to a molecule that has been targeted to the disease in advance of administering $^{227}$Th. Suitable targeting moieties include poly- and oligo-peptides, proteins, DNA and RNA fragments, aptamers etc, preferably a protein, e.g. avidin, strepatavidin, a polyclonal or monoclonal antibody (including IgG and IgM type antibodies), or a mixture of proteins or fragments or constructs of protein. Antibodies, antibody constructs, fragments of antibodies (e.g. FAB fragments), constructs of fragments (e.g. single chain antibodies) or a mixture thereof are particularly preferred.

Also suitable for use in the present invention are therapeutic conjugates of $^{227}$Th with a peptide, amino acid, steroidal or non-steroidal hormone, estrogen, testosterone, biotin, or other specific-binding compounds with molecular weight typically below 10 000 g/mol.

It will thus be appreciated that the soft tissue targeting complexing agent is a bifunctional agent: one moiety must serve to complex the thorium ion, preferably in a chelate complex, i.e. one in which the thorium is multiply complexed; and a further moiety must serve as a vector to target the complex to the soft tissue which is to be treated. The complexing moiety may consist of one or more functional groups present on the targeting moiety or which may be introduced onto the targeting moiety by chemical treatment. More generally however the complexing moiety is conjugated directly or indirectly (e.g. via a linker moiety) to the targeting moiety. Such constructs of active (e.g. therapeutically or diagnostically active) metal - complexing moiety - optional linker moiety - targeting moiety are well known in the fields of targeted radiopharmaceuticals and targeted imaging agents and may be selected and constructed for thorium in analogous fashion. In this regard reference may be had for example to "Handbook of Targeted Delivery of Imaging Agents", Ed. Torchilin, CRC Press, 1995.

Thorium-227 in the present invention will preferably be conjugated to a targeting molecule by using bifunctional chelators. These could be cyclic, linear or branched chelators. Particular reference may be made to the polyaminopolyacid chelators which comprise a linear, cyclic or branched polyazaalkane backbone with acidic (e.g. carboxyalkyl) groups attached at backbone nitrogens. Examples of suitable chelators include DOTA derivatives such as p-isothiocyanatobenzyl-1,4,7,10-tetraazacyclododecane-1,4,7,10-tetraacetic acid (p-SCN-Bz-DOTA) and DTPA derivatives such as p-isothiocyanatobenzyl-diethylenetriaminepentaacetic acid (p-SCN- Bz-DTPA), the first being cyclic chelators, the latter linear chelators.

Derivatives of 1,4,7,10-tetraazacyclododecane-1,4,7,10-tetraacetic acid are particularly preferred chelators for thorium in the present invention, but are not known for their ability to co-ordinate to tetravalent metals such as thorium. It has been the present inventor's surprising finding that although standard methods cannot easily be used to chelate thorium with DOTA derivatives, heating of the DOTA derivative with the metal provides the chelate effectively, as indicated in the examples below. In a further aspect, the present invention thus provides a method for forming a complex of the invention or suitable for use in the methods of the invention comprising heating thorium-227 with a derivative of 1,4,7,10-tetraazacyclododecane-1,4,7,10-tetraacetic acid to form a chelated thorium -227 and subsequently attaching the chelated thorium-227 to a targeting moiety. Any suitable targeting moitely, such as those indicated herein (e.g. below) may be used.

Metallation of the complexing moiety may be performed before or after conjugation of the complexing moiety to the targeting moiety. Where the heating method indicated above is used, however, it is preferable for the metal to be co-ordinated before the attachment of the targeting moiety.

The chelators will preferably be non-phosphonate molecules and in the present invention the $^{227}$Th will preferably not be attached to any phosphonate or other bone-targeting group.

Types of targeting compounds that may be linked to thorium-227 via a chelator include monoclonal or polyclonal antibodies, growth factors, peptides, hormones and hormone analogues, folate and folate derivatives, botin, avidin and streptavidin or analogues thereof. Other possible carriers could be RNA, DNA, or fragments thereof, oligonucleotides, carbohydrates, lipids or compounds made by combinings such groups with or without proteins etc.

The thorium-227 is conjugated to a targeting moiety with bioaffinity, excluding bone-seekers, liposome and folate conjugated antibodies or antibody fragments, to irradiate soft-tissue for therapeutic purposes.

The thorium-227 labeled molecules of the invention may be used for the treatment of cancerous or non-cancerous diseases by targeting disease-related receptors. Typically, such a medical use of $^{227}$Th will be by radioimmuno-therapy based on linking $^{227}$Th by a chelator to an antibody, an antibody fragment, or a construct of antibody or antibody fragments for the treatment of cancerous or non -cancerous diseases. The use of $^{227}$Th in methods and pharmaceuticals according to the present invention is particularly suitable for the treatment of any form of cancer and rheumatological disease, especially cancer of the skin, prostate, cervix, or breast, or inflammatory diseases such as arthritis or fibrositis.

Experiments with thorium labelling of monoclonal antibodies in vitro presented herein have demonstrated that thorium-227 can be bound via a bifunctional chelator to a carrier molecule. It is also demonstrated that such $^{227}$Th immunoconjugates showed specific binding ability towards the CD20 antigen expressing human lymphoma cell line DAUDI and that a relevant number of $^{227}$Th atoms could be bound per cell. It is thereby for the first time shown that receptor targeting with a $^{227}$Th-labeled molecule is feasible.

An interesting feature of the use of thorium-227 is that the radiation intensity will increase with time because of ingrowths of daughter radionuclides, i.e. the dose delivered to normal organs could be kept low during uptake and elimination phases. This is illustrated by Figure 1 of the accompanying drawings. If retention were high in tumor, the dose rate there would increase with time, due to in-growth of daughter nuclides, depending on tumor retention of daughter nuclide. Due to the difficulties of recoil energies, however, efficient retention of daughters at the target site normally requires very specific methods of delivery, such as in liposomes or such that the radionuclide is incorporated into mineralised bone.

The amount of $^{223}$Ra released could be diminished if the molecule carrying $^{227}$Th has a short biological retention half-time in vivo because the radionuclide will mostly be eliminated before a high proportion of the $^{227}$Th has decayed to $^{223}$Ra. The amount of $^{227}$Th would, however, need to be increased in order to remain therapeutically effective, according to the present invention. Preferred biological half-times in vivo are less than 7 days, preferably less than 4 days and particularly less than 2 days. If the complexing agent is selected so as to deliver the $^{227}$Th into the interior of the targeted cells, this will further increase the specific cytotoxicity and reduce the systemic toxic effect of the radioactive daughters because of at least partial retention of daughter isotopes at the tumour site. Both of these features widen the $^{227}$Th therapeutic window and thus form preferred embodiments of the invention.

In a further embodiment of the invention, patients with both soft tissue and skeletal disease may be treated both by the $^{227}$Th and by the $^{223}$Ra generated *in vivo* by the administered thorium. In this particularly advantageous aspect, an extra therapeutic component to the treatment is derived from the acceptably non-myelotoxic amount of $^{223}$Ra by the targeting of the skeletal disease. In this therapeutic method, $^{227}$Th is typically utilised to treat primary and/or metastatic cancer of soft tissue by suitable targeting thereto and the $^{223}$Ra generated from the $^{227}$Th decay is utilised to treat related skeletal disease in the same subject. This skeletal disease may be metastases to the skeleton resulting from a primary soft-tissue cancer, or may be the primary disease where the soft-tissue treatment is to counter a metastatic cancer. Occasionally the soft tissue and skeletal diseases may be unrelated (e.g. the additional treatment of a skeletal disease in a patient with a rheumatological soft-tissue disease).

The invention will now be illustrated by the following non-limiting Examples.

EXAMPLES

*Background Assumption*

Generally the tumor tissue weight in a subject will be low compared to body weight and even if significant concentration and retention of the thorium complex in tumor is obtained, typically 1% or less of the thorium will reach tumor tissue in humans. The soft tissue exposure could therefore be estimated based on whole body clearance of the thorium complex. The effect of tumour targeting will thus be neglected in Examples 1 and 2, which show the influence of biological half-life on the amount of $^{223}$Ra generated *in vivo* relative to the amount of $^{227}$Th administered.

*Materials*

Ammonium acetate (AmAc), L-ascorbic acid (AscA), diethylenetriaminepentaacetic acid (DTPA), ethylenediaminetetraacetic acid (EDTA), sodium carbonate ($Na_2CO_3$), sodium hydrogen carbonate ($NaHCO_3$), tetramethylammo-nium acetate (TMAA, 90% purity) were obtained from Aldrich (Milwaukee, WI, USA) and unless stated exceeded 99% purity. 2-(p-isothiocyanatobenzyl)-1,4,7,10-tetraazacyclododecane (NCS-DOTA) was obtained from Macrocyclics, Dallas, TX, USA. Bovine Serum Albumin (BSA) and Albumine bovine fraction V were obtained from Sigma, St. Louis, MO, USA. Phosphate buffered saline (PBS), foetal bovine serum (FBS) and RPMI 1640 medium with glutamax were obtained from Gibco, Paisley, Scotland, UK. The RPMI 1640 medium was supplied with 15% FBS, penicillin and streptomycin. Anion exchange material was obtained from Bio-Rad Laboratories, Hercules, CA, USA. Mabthera (rituximab) was obtained from F. Hoffmann-La Roche AG, Basel, Switzerland. The cell line used was the CD20 positive lymphoma DAUDI purchased from the European Collection of Cell Cultures (ECACC), Salisbury, UK.

*Example 1. Estimate of the in vivo generation of $^{223}Ra$ following administration of a thorium labeled compound with a whole body retention half-life of 12 hours*

The effective half-life for $^{227}Th$ (assuming negligible fraction of $^{227}Th$ retained in tumor) would be $1/T_{1/2effective}$ = $1/T_{1/2phys}$+$1/T_{1/2biol}$ $\Rightarrow$ $T_{1/2effective}$ = 0.487 days. The fraction of $^{227}Th$ decaying in the body would be equivalent with $T_{1/2\ effective}$ /$T_{1/2\ physical}$ = 0.0262 which would correspond to the generation of 6.1 x $10^9$ atoms of $^{223}Ra$ per 100 kBq of $^{227}Th$ injected. The toxic component from the daughter nuclides should be roughly equivalent to a radium-223 dosage of 4.3 kBq of $^{223}Ra$ per 100 kBq (initial) of $^{227}Th$. Decay of 0.0262 of the administered thorium equivalent to 2.6 kBq of fully retained $^{227}Th$ for every 100 kBq administered.

*Example 2. Estimate of the in vivo generation of $^{223}Ra$ following administration of a thorium labeled compound with a whole body retention half-life of 4 days*

Calculated as in Example 1, the $T_{1/2\ effective}$ = 3.3 days for body clearance. This is equivalent to a fraction of 0.176 of the Th atoms decaying in the body. This corresponds to 4.1 x $10^{10}$ atoms of $^{223}Ra$ generated per 100 kBq of $^{227}Th$. The toxic component from the daughter nuclides should be roughly equivalent to an injected dose of 29 kBq of $^{223}Ra$ per 100 kBq of $^{227}Th$ injected. With this biological half-life, 100 kBq of $^{227}Th$ administered is equivalent to 17.6 kBq fully retained.

*Example 3: Preparation of $^{227}Th$.*

Thorium-227 was selectively isolated from a $^{227}Ac$ mixture, which had been growing in daughters for two weeks, by adding 0.25 ml of 7 M $HNO_3$ to the Ac mixture (which had been evaporated to dryness) and eluting the solution through an anion exchange column. The column had an inner diameter of 2 mm and a length of 30 mm containing approximately 70 mg of AG-1 X 8 anion exchange resin (Biorad Laboratories, Hercules, CA, USA) (nitrate form). The column was washed with 2 -4 ml of 7 M $HNO_3$ to remove $^{227}Ac$, $^{223}Ra$ and Ra daughters while retaining $^{227}Th$. Subse-quently $^{227}Th$ was stripped from the column with a few ml of 12 M HCl. Finally the HCl was evaporated to dryness and the $^{227}Th$ redissolved in 0.2 M HCl.

*Example 4. Thorium-227 labeling of the bifunctional chelator NCS-DOTA.*

Unless otherwise stated chemicals used were from Aldrich (Milwaukee, WI, USA) and had a purity of 99% or better. To 100 $\mu$l of $^{227}Th$ in 0.2 M HCl solution in a half gram vial was added a solution containing 25 $\mu$l $p$-SCN-Benzyl-DOTA (10 mg/ml) (Macrocyclics Inc, Dallas, TX, USA), 20 $\mu$l L-ascorbic acid (150 mg/ml) and 50 $\mu$l tetramethyl ammonium acetate (300 mg/ml) (90% purity) to a pH of about 5.5. The reaction mixture was reacted on a shaker/incubator (Thermomixer Comfort, Eppendorf AG, Hamburg, Germany) at 55 °C for 1 hour. (This would typically cause a quantitative elution of $^{227}Th$ through a 0.5 ml Sephadex C-25 column using 2.5 ml 0.9% NaCl solution, while $^{223}Ra$ (uncomplexed) would be almost quantitatively retained on the column. It was also verified in a control experiment with $^{227}Th$ in a "reaction" solution without the chelator, that both $^{227}Th$ and $^{223}Ra$ were > 90% retained on the column). The unpurified reaction product of $^{227}Th$-p-SCN-Benzyl-DOTA was used for labeling of rituximab.

*Example 5: Preparation of a $^{227}Th$ based radioimmunoconjugate (RIC).*

The labelling was performed via a two-step procedure, the first step being the combination of the $^{227}Th$ and the chelator (described in Example 4). The second step is the coupling of the radioactive chelator to the antibody. The reaction solution (Example 4) was added to 200 µl of rituximab (10 mg /ml, Mabthera®, F. Hoffmann-La Roche AG, Basel, Switzerland) and the reaction solution adjusted to pH - 9 by adding approximately 100 µl of 1 M $Na_2CO_3$/ $NaHCO_3$. The reaction solution was mixed gently on a shaker (Thermomixer Comfort, Eppendorf AG, Hamburg, Germany) at 35 °C for 1 h. Thereafter, 50 µl of 10 mM diethylenetriamine pentaacetic acid (DTPA, Fluka Chemie AG Buchs, Neu-Ulm, Germany) and 200 µl of 0.2 M glycine in saturated borate (sodium tetraborate decahydrate, from Fluka) and the incubation was continued for 5 minutes. Thereafter, the reaction mixture was transferred to a Sephadex G-25 PD 10 column and eluted with 1% BSA (Albumin, Bovine Fraction V, Sigma Chemical Co., St. Louis, MO, USA) in PBS (Gibco, Paisley, Scotland, UK). The eluate was collected in fractions of ~ 0.6 ml which was counted on a dose calibrator (CRC-127R, Capintec, Ramsey, NJ, USA) and the fractions corresponding to the protein eluate was analysed by gamma spectroscopy (GEM15-P detector and Gammavision 5.20 soft-ware, both from EG&G Ortec, Oak Ridge, TN USA) to determine $^{227}$Th gammas vs. $^{223}$Ra gammas, in each fraction before any further use. The following gamma peaks were used: $^{227}$Th; 236.0 keV (11.6 % abundance), 256.3 keV (7.4%), 329.9 keV (2.8%). $^{223}$Ra; 154.2 keV (6.0 %), 269.4 keV (13.6%), 323.9 keV (3.7%) respectively. Fractions 6 and 7 corresponding to about 50% of the protein eluate (as verified by eluting $^{125}$I-labeled rituximab through PD-10 columns) were used because they were essentially free from [223]Ra. Fractions 8 and 9 contained larger amounts of $^{223}$Ra indicating a significant overlap between protein and smaller molecules in these fractions (when re-purified on a PD-10 column these two fractions yielded about 50% of the $^{227}$Th in the 6 and 7 fractions from the new eluate, verifying the presence of $^{227}$Th-rituximab). Based on the measurement of PD-10 eluate fractions on the Ge-detector it is estimated that the overall labeling yield was approximately 12%. It was also shown with a 5 days old preparation stored at 8 °C, that a $^{227}$Th-antibody conjugate could easily be purified on a PD-10 column to remove $^{223}$Ra generated from decay of [227]Th.

It was thus shown that $^{227}$Th could be bound to a targeting molecule via a bifunctional chelator and purified from daughter products. When stored, the generated daughter product $^{223}$Ra would be released from the chelator and by using gel filtration/size exclusion purification, it was possible to regenerate a pure $^{227}$Th-antibody conjugate.

*Example 6: Binding of $^{227}$Th labeled antibody to DAUDI human lymphoma cells.*

DAUDI cells were purchased from European Collection of Cell Cultures (ECACC), Salisbury, UK, and grown according to supplier's instructions using culture medium and supplement from Gibco (Paisley, Scotland, UK) and using 500 ml culture flasks (Cell Star, Greiner Bio-One GmbH, Frickenhausen, Germany). DAUDI cells ($2 \times 10^7$ cells in 0.7 ml PBS) were used to study binding of $^{227}$Th labeled rituximab in vitro. As control of nonspecific binding, DAUDI cells pre-saturated (blocked) with 40 µg unlabeled rituximab for 15 minutes were used. To the test tubes (Polystyrene culture test tubes, 12 × 75 mm, Elkay, Shrewsbury, MA, USA) were added $^{227}$Th labeled rituximab corresponding to 1.3, 5.3, or 26 µg/ml respectively. The experiment was performed in duplicate at each concentration level using unblocked and blocked cells. Incubation was performed for two hours at 8 °C. After incubation, cell suspensions were counted for radioactivity (Crystal II Multidetector, Packard Instrument Company Inc. Downers Grove, IL, USA) and the cells washed with 2 ml of 1% BSA (Albumin, Bovine Fraction V, Sigma Chemical Co., St. Louis, MO, USA) in PBS (Gibco, Paisley, Scotland, UK) and centrifuged (Centrifuge 5810 R, Eppendorf AG, Hamburg, Germany) at 200 rpm for 5 minutes. The washing/centrifugation was repeated twice. Thereafter the cell pellets were counted for radioactivity. The results (each the mean of a duplicate) are set out in Table 3 below.

Table 3

| RIC conc. µg/ml added | Mean cpm bound per cell pellet | | Thorium-227 atoms bound per cell | | |
|---|---|---|---|---|---|
| | Unblocked | Blocked | Unblocked | Blocked | Net specific bound |
| 1.3 | 226 | 25 | 1.9 | 0.2 | 1.7 |
| 5.3 | 1186 | 97 | 10.1 | 0.8 | 9.3 |
| 26.0 | 4141 | 315 | 35.2 | 2.7 | 32.5 |

The results show that $^{227}$Th-labeled rituximab did bind specifically to the DAUDI cells. On average, about 12 times as much RIC bound to the unblocked vs. the blocked cells. Moreover, a therapeutically relevant number of $^{227}$Th atoms were bound per cell.

Thus using a bifunctional chelator useful for attachment to antibodies, peptides and vitamins etc., it was possible to prepare a $^{227}$Th-labeled RIC with the ability to bind a therapeutic relevant number of $^{227}$Th atoms specifically to tumor cells.

*Example 7*: *Estimale of $^{223}$Ra toxicity from prior art*

Because of the lack of human data on toxicity of radium-223 and radium-224, an assumed radiotoxicity for $^{223}$Ra can be derived as follows, using published data for radium-224 in dogs. The decay series (including the decays of daughter nuclides) of both $^{224}$Ra and $^{223}$Ra causes an emission of four alpha panicles per radium atoms. The total alpha particle doses from $^{223}$Ra and $^{224}$Ra in equilibrium with daughters are approximately 26.3 and 27.1 MeV per transformation respectively and are therefore closely equivalent. The half-lives of $^{224}$Ra is 3.62 days and for $^{223}$Ra it is 11.43 days. This means that per activity unit injected, the skeletal dose from $^{223}$Ra would be approximately 3.1 times that from $^{224}$Ra, taking into account alpha energy and half-life differences, and assuming long-term biological retention of the radionuclide (i.e., the clearance is governed by the physical half-life of the radionuclide). This is a valid assumption because Ra is a Ca analogue and is readily incorporated into the skeleton.

The blood cell type most strongly affected after treatment with $^{224}$Ra or $^{223}$Ra appears to be neutrophils. Data from a published study on the biological effects of $^{224}$Ra in adult dogs (see Muggenburg, Radiat Res 146: 171-186, (1996)), administered as a single intravenous injection, show neutrophil cell numbers strongly reduced at 120 and even more so at 350 kBq per kg of bodyweight (kBq/kg), as indicated in Table 4 below.

Table 4.

| Activity administered (kBq/kg) | Days to nadir | % of baseline at nadir | Number of subjects |
|---|---|---|---|
| 13 | 30 | 60 | 12 |
| 40 | 10 | 70 | 12 |
| 120 | 10 | 20 | 6 |
| 350 | 10 | 4 | 8 |

At 350 kBq/kg death, caused by haematological dyscrasia resulting from bone marrow destruction, occurred in some subjects (3 dogs out of 8). It may therefore be assumed that the maximum tolerable dose of $^{224}$Ra is between 120 and 350 kBq/kg in dogs. Translated into $^{223}$Ra, as described above, this would correspond to 39-113 kBq/kg of $^{223}$Ra. Assuming a similar haematological toxicity in dogs and humans, one would expect to reach a maximum tolerable dose within the range of 39 to 113 kBq/kg of $^{223}$Ra in humans. Generally, one has to be careful when comparing data from two different species. However, dogs and humans are very similar with respect to bone marrow toxicity from irradiation (see Hall, "Radiobiology for the radiologist, "Lippincott Williams & Wilkins, Philadelphia, PA, USA, 2000) and thus it would be expected that this calculation would give an effective estimate of the maximum tolerable dose of $^{223}$Ra in humans.

*Example 8: Laboratory study of $^{223}$Ra in humans*

In a phase I study in patients with breast or prostate cancer, dose levels of 37, 74, 130, 170 and 200 kBq/kg of $^{223}$Ra were given as single doses. Neutrophil cell fractions were monitored as a sensitive measure ofhaematological toxicity. The results are set out in Table 5 below.

Table 5.

| Activity administered (kBq/kg) | Days to nadir | ~% of baseline at nadir | Number of subjects |
|---|---|---|---|
| 37 | 14 | 60 | 5 |
| 74 | 14 | 60 | 5 |
| 130 | 28 | 50 | 5 |
| 170 | 21 | 40 | 5 |
| 200 | 21 | 30 | 5 |

It was thus surprisingly found that high dose levels were tolerable in humans and this indicates that it is possible to deliver significantly higher radiation doses to the bone surfaces with $^{223}$Ra than was previously anticipated without causing adverse haematological toxicity.

*Example 9: Further Laboratory study of $^{223}$Ra in humans*

The experiment of Example 8 was carried out with dose metering calibrated to a high accuracy. Dose levels measured at 46, 93, 163, 213, and 250 kBq/kg of $^{223}$Ra were given as single doses and a multiple-dose schedule was also introduced.

*Patients and Study Criteria.*

Thirty-one patients with metastases to the skeleton, 10 from breast- and 21 from prostate cancer, all, were enrolled in phase I A and phase I B trials. The prostate cancer patients were from 60 to 85 years and had body weights ranging from 50 to 120 kg. All of them had progressive disease considered hormone refractory. The breast cancer patients were from 40 to 70 years, with bodyweight from 50 to 95 kg. All of them were progressing second hormonal and/or chemotherapy. The primary objective was to evaluate the safety and tolerance of $^{223}$Ra.

*Follow up.*

The follow up period was 8 weeks.

*Dose Levels and Treatment Schedules.*

In the single injection trial the following average dose levels were used; 46, 93, 163, 213, and 250-kBq kg$^{-1}$ bodyweight (b.w.). Five patients were included at each dose level. Fifteen prostate- and 10 breast cancer patients were included.

In the repeated injection schedule, 6 patients, all with prostate cancer, were included. Three patients were scheduled to receive 5 doses, each of 50 kBq kg$^{-1}$ b.w. at three-weeks intervals, and three patients to receive 2 doses, each of 125 kBq kg$^{-1}$ b.w. at six-weeks intervals.

*Blood Clearance.*

Approximately 1 ml of blood was withdrawn at different time-points post injection and used for radioactivity measurements to determine the blood clearance profiles of the 25 patients included in the single-injection schedule. The weight of each blood sample was determined and the count rate per ml of blood was calculated (assuming 1 ml of blood equals 1 g). The radioactivity was measured in a NaI well type counter. The activity level immediately after injection was calculated assuming that 100% of the activity was initially in the blood and that the total blood weight represented 7% of the body weight. The data are presented as biological data, i.e., adjusted for the radioactive decay between the time of injection and the time of measurement.

*Radionuclide Production.*

Radium-223 was produced from $^{227}$Ac/$^{227}$Th and purified using Ac-resin to immobilize $^{227}$Ac and $^{227}$Th as described in WO0040275. The product concentrate, i.e., dissolved $^{223}$RaCl$_2$ was tested for radionuclide purity by gamma spectroscopy before further use. A concentrate of the $^{223}$Ra in NaCl and Na Citrate was sent sterile production. Isotonicity, pH, and activity concentration were adjusted; a sample kept aside for pathogen and pyrogen testing, and the final product was filled in sterile vials that were subsequently capped with a sealed rubber membrane penetrable to syringes. The vials were put into lead containers and shipped to the hospitals.

*Side-effects.*

Dose-limiting toxicity was not observed in the dose escalating part of the study. Reversible myelosuppression occurred, with nadir 2-3 weeks after injection and recovery during the follow-up period. Neutropenia of maximum grade 3 occurred in two out of the 25 patients. The thrombocytes revealed only grade 1 toxicity, even at the two highest dose levels. In general, there was a slight tendency towards higher myelosuppression with increase in the dose level but the effect was not very pronounced. Few adverse events were seen, nausea being the most frequent one occurring in four out of five patients at the highest dose level. Reversible diarrhoea, grades 1 and 2, responding well to medication, were observed in all dose groups and for a total of approximately 50% of the patients. In the highest dose group vomiting occurred in four of the five patients. This was not observed in the other dose groups.

*Repeated Injection Schedules.*

The three patients in the 50 x 5 schedule did not experience any additional toxicity related to the repetition of the treatment. It appears from the haematological profiles were smoothened out because of the fractionation schedule compared to a single dose totalling the same as the five fractions together.

*Adverse Events Not Related to $^{223}$Ra.*

In the repeated dosing schedule only one of the patients in the 125 x 2 schedule actually got the second dose. Of the two patients not given the follow up dose, one died because of progression in liver metastases, and the other was deemed unfit for further treatment due to recurrence of a previous heart condition.

*Myelotoxicity*

The effect of the radium-223 on neutrophil cell fractions, platelets, white-blood-cell count and Heamoglobin were monitored to give a wide ranging measure of haematological toxicity. The effect on neutrophils and white-blood-cells was most pronounced indicating that these are sensitive markers for myelotoxicity. The results were expressed as the number of patients in the study showing a particular level of toxicity on the CTC Toxicity Grade scale and are set out in Table 6 below.

Table 6.

| Parameters | Toxicity CTC Grade | Single dose / kBq/kg | | | | | Retreatment | Multiple dosing / kBq/kg | |
| --- | --- | --- | --- | --- | --- | --- | --- | --- | --- |
| | | 46 n=5 | 93 n=5 | 163 n=5 | 213 n=5 | 250 n=5 | n=2 | 5 x 50 n=3 | 2 x 125 n=3 |
| Platelets | 0 | 5 | 5 | 4 | 5 | 2 | 2 | 2 | 3 |
| | 1 | 0 | 0 | 1 | 0 | 3 | 0 | 1 | 0 |
| Neutrophile | 0 | 4 | 3 | 3 | 2 | 2 | 2 | 1 | 3 |
| | 1 | 0 | 1 | 0 | 1 | 0 | 0 | 0 | 0 |
| | 2 | 1 | 1 | 1 | 2 | 2 | 0 | 2 | 0 |
| | 3 | 0 | 0 | 1 | 0 | 1 | 0 | 0 | 0 |
| WBC | 0 | 4 | 4 | 3 | 2 | 2 | 2 | 1 | 2 |
| | 1 | 0 | 0 | 0 | 0 | 0 | 0 | 0 | 0 |
| | 2 | 1 | 1 | 1 | 2 | 2 | 0 | 2 | 1 |
| | 3 | 0 | 0 | 1 | 1 | 1 | 0 | 0 | 0 |
| Haemoglobin | 0 | 4 | 2 | 4 | 2 | 2 | 1 | 0 | 0 |
| | 1 | 0 | 2 | 0 | 2 | 3 | 1 | 2 | 1 |
| | 2 | 1 | 1 | 1 | 1 | 0 | 0 | 1 | 2 |

**Claims**

1.    Use of a therapeutically effective quantity of a soft tissue targeting complex of thorium-227 and a complexing agent, in the manufacture of a medicament for the treatment of soft tissue disease in a mammalian subject, wherein said effective quantity is such that in use an acceptably non-myelotoxic quantity of radium-223 is generated *in vivo* by nuclear decay of the administered thorium-227 and wherein the thorium-227 is conjugated to a targeting complexing agent, wherein the complexing agent comprises a targeting moiety with bioaffinity, wherein said targeting moiety is one which serves to target the complex to the soft tissue to be treated, excluding bone-seekers, liposomes and folate conjugated antibodies or antibody fragments and wherein the therapeutically effective quantity of thorium-227 is at least 25 kBq/kg.

2.    Use as claimed in claim 1 wherein said subject is human or canine.

3.    Use as claimed in any one of claims 1 to 3 wherein said therapeutically effective quantity is at least 75 kBq of thorium-227 per kilogram bodyweight.

4. Use as claimed in any of claims 1 to 3 wherein said acceptably non-myelotoxic quantity is less than 300 kBq radium-223 per kilogram bodyweight.

5. Use as claimed in any of claims 1 to 4 wherein said complex comprises chelated thorium-227 linked to a targeting moiety selected from the group of antibodies, antibody constructs, antibody fragments, constructs of antibody fragments and mixtures thereof.

6. Use as claimed in any of claims 1 to 5 wherein said soft tissue disease is a malignant disease.

7. Use as claimed in claim 6 wherein the malignant disease is a disease selected from the group of carcinomas, sarcomas, myelomas, lukemias, lymphomas and mixed type cancers.

8. Use as claimed in any of claims 1 to 8 wherein said medicament is used in a method wherein subject is also treated to combat the myelotoxicity of the radium-223 generated therein.

9. Use as claimed in any of claims 1 to 8, wherein said effective quantity is $D_{add}$ as calculated from formula I below, such that in use an acceptably non-myelotoxic quantity $D_{Ra}$ of radium-223 is generated *in vivo* by nuclear decay of the administered thorium-227;

$$D_{add} = \frac{D_{Ra} \times T_{Th}\left((T_{Bio})^{-1} + (T_{Th})^{-1}\right)}{1.65} \qquad (I)$$

wherein:

$T_{Bio}$ is the biological half-life of said soft tissue targeting complex of thorium-227 and a complexing agent;
$T_{Th}$ is the physical half-life of $^{227}$Th (18.7 days);
$D_{add}$ is the activity of the administered $^{227}$Th complex (kBq/kg) and is is at least 25 kBq/kg; and
$D_{Ra}$ is the acceptably non-myelotoxic amount of $^{223}$Ra;
and further, wherein the thorium-227 is conjugated to a targeting moiety with bioaffinity, excluding bone-seekers, liposomes and folate conjugated antibodies or antibody fragments.

10. Use as claimed in claim 9 wherein $D_{Ra}$ is 200 kBq/kg

11. Use as claimed in any of claims 1 to 10 wherein said medicament is for use in combination with at least one further treatment modality selected from surgery, external beam radiation therapy, chemotherapy, endoradionuclide therapy with radionuclides other than $^{227}$Th, and/or tissue temperature adjustment.

12. A pharmaceutical composition comprising a soft tissue targeting complex of thorium-227 and a complexing agent, together with at least one pharmaceutical carrier or excipient wherein the thorium-227 is conjugated to a targeting complexing agent, wherein the complexing agent has a molecular weight from 100 g/mol to several million g/mol and comprises a targeting moiety with bioaffinity, wherein said targeting moiety is one which serves to target the complex to the soft tissue to be treated, excluding bone-seekers, liposomes and folate conjugated antibodies or antibody fragments and wherein the thorium-227 is present at a therapeutically effective quantity of at least 25 kBq/kg.

13. A soft tissue targeting complex of thorium-227 and a complexing agent wherein the thorium-227 is is chelated by a derivative of 1,4,7,10-tetraazacyclododecane-1,4,7,10-tetraacetic acid and conjugated to a targeting complexing agent, wherein the complexing agent comprises a targeting moiety with bioaffinity, wherein said targeting moiety is one which serves to target the complex to the soft tissue to be treated, excluding bone-seekers, liposomes and folate conjugated antibodies or antibody fragments.

14. A method for forming a complex as claimed in claim 13 comprising heating said thorium-227 with said derivative of 1,4,7,10-tetraazacyclododecane-1,4,7,10-tetraacetic acid to form a chelated thorium-227 and subsequently attaching said chelated thorium-227 to a targeting moiety.

15. A kit for use in a method for the treatment of soft tissue disease in a mammalian subject, said kit comprising

a) a solution of a soft tissue targeting complex of thorium-227 and a complexing agent wherein the thorium-227 is conjugated to a targeting complexing agent, wherein the complexing agent has a molecular weight from 100 g/mol to several million g/mol and comprises a targeting moiety with bioaffinity, wherein said targeting moiety is one which serves to target the complex to the soft tissue to be treated, excluding bone-seekers, liposomes and folate conjugated antibodies or antibody fragments, and

b) instructions for the use of said solution in said method.

16. A therapeutically effective quantity of a soft tissue targeting complex of thorium-227 and a complexing agent, for use in the treatment of soft tissue disease in a mammalian subject, wherein said effective quantity is such that in use an acceptably non-myelotoxic quantity of radium-223 is generated *in vivo* by nuclear decay of the administered thorium-227 and wherein the thorium-227 is conjugated to a targeting complexing agent, wherein the complexing agent comprises a targeting moiety with bioaffinity, wherein said targeting moiety is one which serves to target the complex to the soft tissue to be treated, excluding bone-seekers, liposomes and folate conjugated antibodies or antibody fragments and wherein the therapeutically effective quantity of thorium-227 is at least 25 kBq/kg.

**Patentansprüche**

1. Verwendung einer therapeutisch wirksamen Menge eines Weichgewebe-Targetingkomplexes von Thorium-227 und eines Komplexbildners zur Herstellung eines Arzneimittels zur Behandlung einer Erkrankung des Weichgewebes in einem Säugetier, wobei die wirksame Menge derart ist, dass bei Verwendung eine verträgliche nicht-myelotoxische Menge an Radium-223 *in vivo* durch nuklearen Zerfall des verabreichten Thorium-227 erzeugt wird und wobei das Thorium-227 an einen Targeting-Komplexbildner konjugiert ist, wobei der Komplexbildner eine Targeting-Einheit mit Bioaffinität umfasst, wobei die Targeting-Einheit so beschaffen ist, dass sie dazu dient, den Komplex zu dem zu behandelnden Weichgewebe zu leiten, ausgenommen Knochensucher, Liposomen und Folat-konjugierte Antikörper oder Antikörperfragmente und wobei die therapeutisch wirksame Menge von Thorium-227 mindestens 25 kBq/kg ist.

2. Verwendung wie in Anspruch 1 beansprucht, wobei das Säugetier ein Mensch oder ein Hund ist.

3. Verwendung wie in einem der Ansprüche 1 bis 3 beansprucht, wobei die therapeutisch wirksame Menge mindestens 75 kBq Thorium-227 pro Kilogramm Körpergewicht ist.

4. Verwendung wie in einem der Ansprüche 1 bis 3 beansprucht, wobei die verträgliche nicht-myelotoxische Menge weniger als 300 kBq Radium-223 pro Kilogramm Körpergewicht ist.

5. Verwendung wie in einem der Ansprüche 1 bis 4 beansprucht, wobei der Komplex chelatiertes Thorium-227 umfasst, das an eine Targeting-Einheit gebunden ist, die ausgewählt wird aus der Gruppe von Antikörpern, Antikörperkonstrukten, Antikörperfragmenten, Konstrukten von Antikörperfragmenten und Gemischen davon.

6. Verwendung wie in einem der Ansprüche 1 bis 5 beansprucht, wobei die Erkrankung des Weichgewebes eine bösartige Erkrankung ist.

7. Verwendung wie in Anspruch 6 beansprucht, wobei die bösartige Erkrankung eine Erkrankung ist, die ausgewählt wird aus der Gruppe von Karzinomen, Sarkomen, Myelomen, Leukämien, Lymphomen und Krebsarten gemischten Typs.

8. Verwendung wie in einem der Ansprüche 1 bis 8 beansprucht, wobei das Arzneimittel in einem Verfahren verwendet wird, bei dem das Säugetier ebenfalls behandelt wird, um die Myelotoxizität des darin erzeugten Radium-223 zu bekämpfen.

9. Verwendung wie in einem der Ansprüche 1 bis 8 beansprucht, wobei die wirksame Menge $D_{add}$ ist wie aus der unten stehenden Formel I berechnet, so dass bei Verwendung eine verträgliche nicht-myelotoxische Menge $D_{Ra}$ von Radium-223 *in vivo* durch nuklearen Zerfall des verabreichten Thorium-227 erzeugt wird;

$$D_{add} = \frac{D_{Ra} \times T_{th}\{(T_{bio})^{-1} + (T_{Th})^{-1}\}}{1,65} \quad (I)$$

wobei:

$T_{Bio}$ die biologische Halbwertszeit des Weichgewebe Targeting-Komplexes von Thorium-227 und eines Komplexbildners ist;

$T_{Th}$ die physikalische Halbwertszeit von $^{227}$Th ist (18,7 Tage);

$D_{add}$ die Aktivität des verabreichten $^{227}$Th Komplexes (kBq/kg) ist und mindestens 25 kBq/kg beträgt; und

$D_{Ra}$ die verträgliche nicht-myelotoxische Menge von $^{223}$Ra ist;

und ferner, wobei das Thorium-227 an eine Targeting-Einheit mit Bioaffinität konjugiert ist, ausgenommen Knochensucher, Liposomen und Folat-konjugierte Antikörper oder Antikörperfragmente.

10. Verwendung wie in Anspruch 9 beansprucht, wobei $D_{Ra}$ 200 kBq/kg ist.

11. Verwendung wie in einem der Ansprüche 1 bis 10 beansprucht, wobei das Arzneimittel zur Verwendung ist in Kombination mit mindestens einer weiteren Behandlungsweise ausgewählt aus Operation, externer Bestrahlungstherapie, Chemotherapie, Endoradionuklidtherapie mit anderen Radionukliden als $^{227}$Th und/oder Gewebstemperaturanpassung.

12. Pharmazeutische Zusammensetzung, die einen Weichgewebe Targeting-Komplex von Thorium-227 und einen Komplexbildner umfasst gemeinsam mit mindestens einem pharmazeutischen Träger oder Hilfsstoff, wobei das Thorium-227 an einen Targeting-Komplexbildner konjugiert ist, wobei der Komplexbildner ein Molekulargewicht von 100 g/mol bis mehrere Millionen g/mol aufweist und eine Targeting-Einheit mit Bioaffinität umfasst, wobei die Targeting-Einheit so beschaffen ist, dass sie dazu dient, den Komplex zu dem zu behandelnden Weichgewebe zu leiten, ausgenommen Knochensucher, Liposomen und Folat-konjugierte Antikörper oder Antikörperfragmente und wobei das Thorium-227 in einer therapeutisch wirksamen Menge von mindestens 25 kBq/kg vorliegt.

13. Weichgewebe Targeting-Komplex von Thorium-227 und einem Komplexbildner, wobei das Thorium-227 durch ein Derivat von 1,4,7,10-Tetraazacyclododecan-1,4,7,10-tetraessigsäure chelatiert und an einen Targeting-Komplexbildner konjugiert ist, wobei der Komplexbildner eine Targeting-Einheit mit Bioaffinität umfasst, wobei die Targeting-Einheit so beschaffen ist, dass sie dazu dient, den Komplex zu dem zu behandelnden Weichgewebe zu leiten, ausgenommen Knochensucher, Liposomen und Folat-konjugierte Antikörper oder Antikörperfragmente.

14. Verfahren zur Bildung eines Komplexes wie in Anspruch 13 beansprucht, welches das Erhitzen des Thorium-227 mit dem Derivat von 1,4,7,10-Tetraazacyclododecan-1,4,7,10-tetraessigsäure umfasst zur Bildung eines chelatierten Thorium-227 und daraufhin das Befestigen des chelatierten Thorium-227 an der Targeting-Einheit.

15. Kit zur Verwendung in einem Verfahren zur Behandlung einer Weichgewebserkrankung bei einem Säugetier, wobei der Kit

a) eine Lösung eines Weichgewebe Targeting-Komplexes von Thorium-227 und eines Komplexbildners umfasst, wobei das Thorium-227 an einen Targeting-Komplexbildner konjugiert ist, wobei der Komplexbildner ein Molekulargewicht von 100 g/mol bis mehrere Millionen g/mol aufweist und eine Targeting-Einheit mit Bioaffinität umfasst, wobei die Targeting-Einheit so beschaffen ist, dass sie dazu dient, den Komplex zu dem zu behandelnden Weichgewebe zu leiten, ausgenommen Knochensucher, Liposomen und Folat-konjugierte Antikörper oder Antikörperfragmente, und

b) Anweisungen zur Verwendung der Lösung in dem Verfahren.

16. Therapeutisch wirksame Menge eines Weichgewebe Targeting-Komplexes von Thorium-227 und eines Komplexbildners zur Behandlung einer Erkrankung des Weichgewebes in einem Säugetier, wobei die wirksame Menge derart ist, dass bei Verwendung eine verträgliche nicht-myelotoxische Menge an Radium-223 *in vivo* durch nuklearen Zerfall des verabreichten Thorium-227 erzeugt wird und wobei das Thorium-227 an einen Targeting-Komplexbildner konjugiert ist, wobei der Komplexbildner eine Targeting-Einheit mit Bioaffinität umfasst, wobei die Targeting-Einheit so beschaffen ist, dass sie dazu dient, den Komplex zu dem zu behandelnden Weichgewebe zu leiten, ausgenommen Knochensucher, Liposomen und Folat-konjugierte Antikörper oder Antikörperfragmente und wobei die therapeutisch wirksame Menge von Thorium-227 mindestens 25 kBq/kg ist.

**Revendications**

1. Utilisation d'une quantité efficace d'un point de vue thérapeutique d'un complexe ciblant les tissus mous de thorium 227 et d'un agent complexant, dans la fabrication d'un médicament pour le traitement de maladie des tissus mous chez un sujet mammifère, dans laquelle ladite quantité efficace est telle que, lors de l'utilisation, une quantité non myélotoxique de façon acceptable de radium 223 est produite *in vivo* par désintégration nucléaire du thorium 227 administré et dans laquelle le thorium 227 est conjugué à un agent complexant ciblant, dans lequel l'agent complexant comprend une fraction de ciblage avec une affinité biologique, dans laquelle ladite fraction de ciblage est une fraction qui sert à cibler le complexe vers les tissus mous à traiter, excluant les substances ostéotropes, les liposomes et des fragments d'anticorps ou des anticorps conjugués avec du folate et dans laquelle la quantité efficace d'un point de vue thérapeutique de thorium 227 est d'au moins 25 kBq/kg.

2. Utilisation selon la revendication 1, dans laquelle ledit sujet est humain ou canin.

3. Utilisation selon l'une quelconque des revendications 1 à 3, dans laquelle ladite quantité efficace d'un point de vue thérapeutique est d'au moins 75 kBq de thorium 227 par kilogramme de poids corporel.

4. Utilisation selon l'une quelconque des revendications 1 à 3, dans laquelle ladite quantité non myélotoxique de façon acceptable est inférieure à 300 kBq de radium 223 par kilogramme de poids corporel.

5. Utilisation selon l'une quelconque des revendications 1 à 4, dans laquelle ledit complexe comprend du thorium 227 chélaté lié à une fraction de ciblage sélectionnée à partir du groupe d'anticorps, de constituants d'anticorps, de fragments d'anticorps, de constituants de fragments d'anticorps et de mélanges de ceux-ci.

6. Utilisation selon l'une quelconque des revendications 1 à 5, dans laquelle ladite maladie des tissus mous est une maladie maligne.

7. Utilisation selon la revendication 6, dans laquelle la maladie maligne est une maladie sélectionnée à partir du groupe des carcinomes, des sarcomes, des myélomes, des leucémies, des lymphomes et des formations cancéreuses de type mixte.

8. Utilisation selon l'une quelconque des revendications 1 à 8, dans laquelle ledit médicament est utilisé dans un procédé dans lequel le sujet est également traité pour combattre la myélotoxicité du radium 223 produit en son sein.

9. Utilisation selon l'une quelconque des revendications 1 à 8, dans laquelle ladite quantité efficace est $D_{add}$ telle que calculée à partir de la formule I ci-dessous, de sorte que, lors de l'utilisation, une quantité non myélotoxique de façon acceptable $D_{Ra}$ de radium 223 est produite *in vivo* par désintégration nucléaire du thorium 227 administré ;

$$D_{add} = \frac{D_{Ra} \times T_{Th}\left((T_{Bio})^{-1} + (T_{Th})^{-1}\right)}{1.65} \qquad (I)$$

dans laquelle :

$T_{Bio}$ est la demi-vie biologique dudit complexe de thorium 227 et d'un agent complexant ciblant les tissus mous ;
$T_{Th}$ est la demi-vie physique de $^{227}$Th (18,7 jours) ;
$D_{add}$ est l'activité du complexe $^{227}$Th administré (kBq/kg) et est d'au moins 25 kBq/kg ; et
$D_{Ra}$ est la quantité non myélotoxique de façon acceptable de $^{223}$Ra ;
et en outre, dans laquelle le thorium 227 est conjugué à une fraction de ciblage avec une affinité biologique, excluant les substances ostéotropes, les liposomes et des fragments d'anticorps ou des anticorps conjugués à du folate.

**10.** Utilisation selon la revendication 9, dans laquelle $D_{Ra}$ est 200 kBq/kg

**11.** Utilisation selon l'une quelconque des revendications 1 à 10, dans laquelle ledit médicament est pour une utilisation en combinaison avec au moins une modalité de traitement supplémentaire sélectionnée à partir de la chirurgie, thérapie par rayonnement de faisceau externe, chimiothérapie, endoradiothérapie avec des radionucléides autres que $^{227}$Th, et/ou ajustement de température de tissu.

**12.** Composition pharmaceutique comprenant un complexe de thorium 227 et d'un agent complexant ciblant les tissus mous, en même temps qu'au moins un vecteur ou un excipient pharmaceutique dans lequel le thorium 227 est conjugué à un agent complexant ciblant, dans lequel l'agent complexant a une masse moléculaire de 100 g/mol à plusieurs millions de g/mol et comprend une fraction de ciblage avec une affinité biologique, dans laquelle ladite fraction de ciblage est une fraction qui sert à cibler le complexe vers les tissus mous à traiter, excluant les substances ostéotropes, les liposomes et des fragments d'anticorps ou des anticorps conjugués avec du folate et dans laquelle le thorium 227 est présent en une quantité efficace d'un point de vue thérapeutique d'au moins 25 kBq/kg.

**13.** Complexe de thorium 227 et d'un agent complexant ciblant les tissus mous dans lequel le thorium 227 est chélaté par un dérivé d'acide 1,4,7,10-tétraazacyclododécane-1,4,7,10-tétra-acétique et conjugué à un agent complexant ciblant, dans lequel l'agent complexant comprend une fraction de ciblage avec une affinité biologique, dans lequel ladite fraction de ciblage est une fraction qui sert à cibler le complexe vers les tissus mous à traiter, en excluant les substances ostéotropes, les liposomes et des fragments d'anticorps ou des anticorps conjugués avec du folate.

**14.** Procédé pour former un complexe selon la revendication 13 comprenant le chauffage dudit thorium 227 avec ledit dérivé d'acide 1,4,7,10-tétraazacyclododécane-1,4,7,10-tétra-acétique pour former un thorium 227 chélaté et par la suite attacher ledit thorium 227 chélaté à une fraction de ciblage.

**15.** Kit à utiliser dans un procédé pour le traitement de maladie des tissus mous chez un sujet mammifère, ledit kit comprenant a) une solution d'un complexe de thorium 227 et d'un agent complexant ciblant les tissus mous dans lequel le thorium 227 est conjugué à un agent complexant ciblant, dans lequel l'agent complexant a une masse moléculaire allant de 100 g/mol à plusieurs millions de g/mol et comprend une fraction de ciblage avec une affinité biologique, dans laquelle ladite fraction de ciblage est une fraction qui sert à cibler le complexe vers les tissus mous à traiter, excluant les substances ostéotropes, les liposomes et des fragments d'anticorps ou des anticorps conjugués avec du folate, et b) des instructions pour l'utilisation de ladite solution dans ledit procédé.

**16.** Quantité efficace d'un point de vue thérapeutique d'un complexe de thorium 227 et d'un agent complexant ciblant les tissus mous, à utiliser dans le traitement de maladie des tissus mous chez un sujet mammifère, dans laquelle ladite quantité efficace est telle que, lors de l'utilisation, une quantité non myélotoxique de façon acceptable de radium 223 est produite *in vivo* par désintégration nucléaire du thorium 227 administré et dans laquelle le thorium 227 est conjugué à un agent complexant ciblant, dans lequel l'agent complexant comprend une fraction de ciblage avec une affinité biologique, dans laquelle ladite fraction de ciblage est une fraction qui sert à cibler le complexe vers les tissus mous à traiter, excluant les substances ostéotropes, les liposomes et des fragments d'anticorps ou des anticorps conjugués avec du folate et dans laquelle la quantité efficace d'un point de vue thérapeutique de thorium 227 est d'au moins 25 kBq/kg.

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0166155 A
- WO 0160417 A
- WO 0205859 A
- US 2001008625 A
- WO 0040275 A

### Non-patent literature cited in the description

- **FEINENDEGEN et al.** *Radiat Res,* 1997, vol. 148, 195-201
- **WILBUR.** *Antibody Immunocon Radiopharm,* 1991, vol. 4, 85-96
- **HALL.** Radiobiology for the radiologist. Lippincott Williams & Wilkins, 2000
- **MAUSNER.** *Med Phys,* 1993, vol. 20, 503-509
- **MÜLLER.** *Int J Radiat Biol,* vol. 20, 233-243
- Alpha-emitters for medical therapy. **FEINENDEGEN et al.** Second BiAnnual Workshop. US Dept. of Energy Report No. DOE/NE-0116, June 1998
- **MÜLLER.** *Int. J. Radiat. Biol.,* 1971, vol. 20, 233-243
- **MUGGENBURG.** *Radiat Res,* 1996, vol. 146, 171-186